# EUROPEAN PATENT APPLICATION

(11) **EP 4 206 326 A1**
(43) Date of publication of application: **05.07.2023**
(21) Application number: 21861679.5
(22) Date of filing: 26.08.2021
(51) Int. Cl.: C12N 15/55, C12N 1/15, C12N 1/19, C12N 1/21, C12N 5/10, C12N 9/14, C12N 15/63, C12P 17/06

(54) **ENZYME DEHYDROXYLATING HYDROXYL GROUP AT 9-POSITION OF UROLITHIN COMPOUND**

(30) Priority: 28.08.2020 JP 2020144115
(71) Applicant: Daicel Corporation, Osaka 530-0011 (JP)
(72) Inventor: ISHIWA, Shunsuke, Tokyo 108-8230 (JP); YAMAMOTO, Hiroaki, Tokyo 108-8230 (JP); OGAWA, Jun, Kyoto-shi, Kyoto 606-8501 (JP); KISHINO, Shigenobu, Kyoto-shi, Kyoto 606-8501 (JP)
(74) Representative: Grünecker Patent- und Rechtsanwälte PartG mbB
(86) International application number: PCT/JP2021/031386
(87) International publication number: WO 2022/045257

(57) **Abstract**

An object of the present disclosure is at least to provide an enzyme that dehydroxylates a hydroxyl group at a 9-position of urolithins having a hydroxyl group at the 9-position, and the object is solved by an enzyme having properties (1) and (2) below: (1) The enzyme dehydroxylates a hydroxyl group at a 9-position of urolithins; and (2) the enzyme is activated by reduced nicotinamide adenine dinucleotide (NADH) and/or reduced nicotinamide adenine dinucleotide phosphate (NADPH).

## Description

### TECHNICAL FIELD

The present disclosure relates to an enzyme that dehydroxylates a hydroxyl group at a 9-position of urolithins.

### BACKGROUND ART

Urolithins such as urolithin A and urolithin C are known as intestinal metabolites of ellagic acid derived from, *inter alia,* ellagitannins included in pomegranates, raspberries, blackberries, cloudberries, strawberries, walnuts, and the like.

For synthesizing these urolithins, a method has been reported in which 2-bromo-5-methoxybenzoic acid is used as a starting material and converted to 2-bromo-5-hydroxybenzoic acid through demethylation, which is then reacted with resorcinol to form urolithin A (Non-Patent Literature 1). However, this type of chemical synthesis is not suitable in use involving urolithins as part of the materials for functional food products, including beverages and supplements.

It is known that, after ellagitannins and ellagic acids are consumed, those are metabolized by intestinal microflora and converted into urolithins. In recent years, a microorganism belonging to the species *Gordonibacter urolithinfaciens* and a microorganism belonging to the species *Gordonibacter pamelaeae* have been discovered as enterobacteria that produce urolithin C, which is a type of urolithin, from ellagic acid, and a method has been reported in which these enterobacteria are used to produce urolithin C through fermentation of ellagic acid (Patent Document 1 and Non-Patent Literature 1).

### CITATION LIST

### PATENT DOCUMENT

Patent Document 1: WO 2014/147280

### NON-PATENT LITERATURE

Non-Patent Literature 1: J. Agric. Food Chem., 56, 393-400 (2008)

### SUMMARY OF INVENTION

### TECHNICAL PROBLEM

An object of the present disclosure is at least to provide an enzyme that dehydroxylates a hydroxyl group at a 9-position of urolithins having a hydroxyl group at the 9-position.

### SOLUTION TO PROBLEM

The present inventors discovered, by proteome analysis of a specific strain of *Clostridium bolteae,* presence of proteins with significantly higher expression level in a case where the strain is cultured in a medium containing urolithin C than in a case where the strain is cultured in a medium not containing urolithin C. The present disclosure includes the following aspects.
<1> An enzyme having properties (1) and (2) below:
   (1) The enzyme dehydroxylates a hydroxyl group at a 9-position of urolithins; and
   (2) the enzyme is activated by reduced nicotinamide adenine dinucleotide (NADH) and/or reduced nicotinamide adenine dinucleotide phosphate (NADPH).
<2> The enzyme according to <1>, having properties (3) to (5) below:
   (3) an SDS-PAGE result includes a band indicating a relative molecular mass of 77,000 or greater and 95,000 or less;
   (4) an optimum pH is 6.0 or higher and 8.5 or lower; and
   (5) an optimum temperature is 37°C or higher and 60°C or lower.
<3> The enzyme according to <1> or <2>, derived from a microorganism belonging to the genus *Clostridium.*
<4> The enzyme according to <3>, wherein the microorganism belonging to the genus *Clostridium* is one or more selected from the group consisting of a microorganism belonging to *Clostridium bolteae,* a microorganism belonging to *Clostridium asparagiforme,* a microorganism belonging to *Clostridium citroniae,* and a microorganism belonging to *Clostridium sp.*
<5> The enzyme according to <4>, wherein
   the microorganism belonging to *Clostridium bolteae* is one or more selected from the group consisting of *Clostridium bolteae* JCM 12243 strain, *Clostridium bolteae* DSM 29485 strain, and *Clostridium bolteae* DSM 15670 strain;
   the microorganism belonging to *Clostridium asparagiforme* is *Clostridium asparagiforme* DSM 15981 strain;
   the microorganism belonging to *Clostridium citroniae* is *Clostridium citroniae* DSM 19261 strain; and
   the microorganism belonging to *Clostridium sp.* is *Clostridium sp.* DC3656 (NITE BP-02708) strain.
<6> The enzyme according to any of <1> to <5>,
   containing an amino acid sequence represented by SEQ ID NO: 3;
   containing an amino acid sequence represented by SEQ ID NO: 6; or
   containing an amino acid sequence represented by SEQ ID NO: 9.
<7> The enzyme according to any of <1> to <6>,
   containing an amino acid sequence represented by SEQ ID NO: 1, an amino acid sequence represented by SEQ ID NO: 2, and an amino acid sequence represented by SEQ ID NO: 3;
   containing an amino acid sequence represented by SEQ ID NO: 4, an amino acid sequence represented by SEQ ID NO: 5, and an amino acid sequence represented by SEQ ID NO: 6; or
   containing an amino acid sequence represented by SEQ ID NO: 7, an amino acid sequence represented by SEQ ID NO: 8, and an amino acid sequence represented by SEQ ID NO: 9.
<8> A polynucleotide,
   containing a base sequence represented by SEQ ID NO: 12,
   containing a base sequence represented by SEQ ID NO: 15; or
   containing a base sequence represented by SEQ ID NO: 18.
<9> A polynucleotide,
   containing a base sequence represented by SEQ ID NO: 10, a base sequence represented by SEQ ID NO: 11, and a base sequence represented by SEQ ID NO: 12;
   containing a base sequence represented by SEQ ID NO: 13, a base sequence represented by SEQ ID NO: 14, and a base sequence represented by SEQ ID NO: 15; or
   containing a base sequence represented by SEQ ID NO: 16, a base sequence represented by SEQ ID NO: 17, and a base sequence represented by SEQ ID NO: 18.
<10> A recombinant vector containing the polynucleotide according to <8> or <9>.
<11> A transformant, retaining the polynucleotide according to <8> or <9> in a manner that the polynucleotide can be expressed or retaining the vector according to < 10> in a manner that the vector can be expressed.
<12> A method for producing a protein encoded by the polynucleotide according to <8> or <9>, the method including culturing the transformant according to <11>.
<13> A method for dehydroxylating a hydroxyl group at a 9-position of urolithins, the method including step (I) below:
   (I) bringing one or more selected from (i) to (iv) below into contact with urolithins having a hydroxyl group at a 9-position to dehydroxylate the hydroxyl group at the 9-position:
      (i) the enzyme according to any of <1> to <7>;
      (ii) a protein encoded by the polynucleotide according to <8> or <9>;
      (iii) a microorganism producing the enzyme described in (i) above or the protein described in (ii) above; and
      (iv) a treated product of the microorganism described in (iii) above.
<14> The method according to <13>, wherein
   the urolithins are urolithin M5, urolithin M6, urolithin C, or isourolithin A; and
   products produced by the dehydroxylation of the hydroxyl group at the 9-position of the urolithins are urolithin E, urolithin M7, urolithin A, and urolithin B, respectively.
<15> A method for producing urolithin A, the method including steps (I) and (II) below:
   (I) allowing a microorganism having an ability to produce urolithin C from ellagic acid to produce urolithin C from ellagic acid;
   (II) bringing one or more selected from (i) to (iv) below into contact with the urolithin C to produce urolithin A:
      (i) the enzyme according to any of <1> to <7>;
      (ii) a protein encoded by the polynucleotide according to <8> or <9>;
      (iii) a microorganism producing the enzyme described in (i) above or the protein described in (ii) above; and
      (iv) a treated product of the microorganism described in (iii) above.
<16> A method for producing urolithin B, the method including steps (I) to (III) below:
   (I) allowing a microorganism having an ability to produce urolithin C from ellagic acid to produce urolithin C from ellagic acid;
   (II) allowing a microorganism having an ability to produce isourolithin A from urolithin C to produce isourolithin A from the urolithin C; and
   (III) bringing one or more selected from (i) to (iv) below into contact with the isourolithin A to produce urolithin B:
      (i) the enzyme according to any of <1> to <7>;
      (ii) a protein encoded by the polynucleotide according to <8> or <9>;
      (iii) a microorganism producing the enzyme described in (i) above or the protein described in (ii) above; and
      (iv) a treated product of the microorganism described in (iii) above.

### ADVANTAGEOUS EFFECTS OF INVENTION

The present disclosure can achieve at least an effect of providing the enzyme that dehydroxylates a hydroxyl group at the 9-position of urolithins having a hydroxyl group at the 9-position.

### BRIEF DESCRIPTION OF DRAWINGS

FIG. 1 is a photograph (a photograph substituted for a drawing) showing results of SDS-PAGE in an experimental example of the present disclosure.
FIG. 2 is a graph showing temperature dependence of an enzyme in an experimental example of the present disclosure.
FIG. 3 is a graph showing pH dependence of an enzyme in an experimental example of the present disclosure.
FIG. 4 is a graph showing temperature stability of an enzyme in an experimental example of the present disclosure.
FIG. 5 is a graph showing pH stability of an enzyme in an experimental example of the present disclosure.
FIG. 6 is a photograph (a photograph substituted for a drawing) showing results of SDS-PAGE in an experimental example of the present disclosure.

### DESCRIPTION OF EMBODIMENTS

Hereinafter, the present disclosure will be described in detail.

Note that each of configurations, their combinations, and the like in each aspect are examples, and additions, omissions, substitutions, and other changes of the configuration can be appropriately made within a scope that does not depart from the spirit of the present disclosure. The present disclosure is not limited by the aspects and is limited only by the scope of the claims.

Each aspect disclosed in the present specification can be combined with any other feature disclosed herein.

Note that in the present disclosure, the accession No. of a strain beginning with the description DSM is the number assigned to a microorganism stored at Deutsche Sammlung von Mikroorganismen und Zellkulturen GmbH (DSMZ).

In addition, the accession No. of a strain beginning with the description JCM is the number assigned to a microorganism stored at the RIKEN BioResource Research Center.

Urolithins are represented by the following General Formula (1): 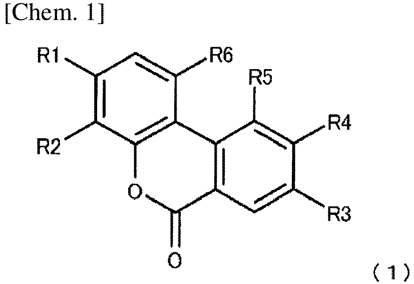 where in the formula, R1 to R6 each independently represent a hydroxyl group, a hydrogen atom, or a methoxy group, and one or more of R1 to R6 are hydroxyl groups.

The hydroxyl group at the 9-position of the urolithins described in the present specification refers to the hydroxyl group of urolithins in which R4 in General Formula (1) above is a hydroxyl group.

### < 1. Enzyme dehydroxylating hydroxyl group at 9-position of urolithins>

One aspect of the present disclosure is an enzyme that dehydroxylates the hydroxyl group at the 9-position of urolithins. In the present specification, this may be referred to as "the enzyme according to the present aspect" or the like. The enzyme is presumed to be composed of a first subunit, a second subunit, or a third subunit.

The enzyme according to the present aspect is an enzyme having properties (1) and (2) below:
(1) the enzyme dehydroxylates a hydroxyl group at the 9-position of urolithins; and
(2) the enzyme is activated by reduced nicotinamide adenine dinucleotide (NADH) and/or reduced nicotinamide adenine dinucleotide phosphate (NADPH).

The urolithins in the present aspect are preferably urolithin M5, urolithin M6, urolithin C, or urolithin A. Dehydroxylation of the hydroxyl group at the 9-position of those by the enzyme according to the present aspect produces urolithin E, urolithin M7, urolithin A, and urolithin B, respectively.

The enzyme according to the present aspect is activated by a cofactor. The cofactor is exemplified by reduced nicotinamide adenine dinucleotide (NADH) and/or reduced nicotinamide adenine dinucleotide phosphate (NADPH). In addition, flavin mononucleotide (FMN) may be used as the cofactor in an embodiment where it is used in combination with NADH and/or NADPH.

The enzyme according to the present aspect preferably has properties (3) to (5) below.
(3) An SDS-PAGE result includes a band indicating a relative molecular mass of preferably 77,000 or greater, more preferably 80,000 or greater, and even more preferably 83,000 or greater, and on the other hand, includes a band indicating a relative molecular mass of preferably 95,000 or less, more preferably 92,000 or less, and even more preferably 89,000 or less.
(4) An optimum pH is preferably 6.0 or higher, more preferably 6.5 or higher, and even more preferably 7.0 or higher, and on the other hand, preferably 8.5 or lower and more preferably 8.0 or lower.
(5) An optimum temperature is preferably 37°C or higher, more preferably 45°C or higher, and even more preferably 50°C or higher, and on the other hand, preferably 60°C or lower, more preferably 58°C or lower, and even more preferably 56°C or lower.

In addition, the enzyme according to the present aspect preferably further has a property (3-1) below:
(3-1) An SDS-PAGE result includes a band indicating a relative molecular mass of preferably 25,000 or greater, more preferably 27,000 or greater, and even more preferably 29,000 or greater, and on the other hand, includes a band indicating a relative molecular mass of preferably 37,000 or less, more preferably 35,000 or less, and even more preferably 33,000 or less.

Furthermore, the enzyme according to the present aspect preferably further has a property (3-2) below:
(3-2) An SDS-PAGE result includes a band indicating a relative molecular mass of preferably 14,000 or greater, more preferably 15,000 or greater, and even more preferably 16,000 or greater, and on the other hand, includes a band indicating a relative molecular mass of preferably 21,000 or less, more preferably 20,000 or less, and even more preferably 19,000 or less.

The enzyme according to the present aspect is preferably derived from a microorganism belonging to the genus *Clostridium.*

The microorganism is more preferably one or more selected from the group consisting of a microorganism belonging to *Clostridium bolteae,* a microorganism belonging to *Clostridium asparagiforme,* a microorganism belonging to *Clostridium citroniae,* and a microorganism belonging to *Clostridium sp.*

A preferred microorganism among microorganisms belonging to the genus *Clostridium bolteae* is one or more selected from the group consisting of a microorganism belonging to *Clostridium bolteae* JCM 12243 strain, *Clostridium bolteae* DSM 29485 strain, and *Clostridium bolteae* DSM 15670 strain.

A preferred microorganism belonging to *Clostridium asparagiforme* is *Clostridium asparagiforme* DSM 15981 strain.

A preferred microorganism belonging to *Clostridium citroniae* is *Clostridium citroniae* DSM 19261 strain.

A preferred *Clostridium sp.* is *Clostridium sp.* DC3656 (NITE BP-02708) strain.

The *Clostridium sp.* DC 3656 (NITE BP-02708) strain was deposited in Japan with Patent Microorganisms Depositary, National Institute of Technology and Evaluation (NITE) (#122, 2-5-8 Kazusa Kamatari, Kisarazu-shi, Chiba-ken, 292-0818) under accession No. NITE P-02708 as of May 8, 2018. Thereafter, a request for transfer of the strain to an international depository under the Budapest Treaty was made as of July 15, 2020 to NITE Patent Microorganisms Depositary, National Institute of Technology and Evaluation [address: #122, 2-5-8 Kazusa Kamatari, Kisarazu-shi, Chiba-ken, 292-0818, Japan], and the accession No. NITE BP-02708 was assigned to the strain.

The *Clostridium bolteae* JCM 12243 strain is not limited to the same strain and may be a bacterium substantially equivalent to the deposited strain. The term "the bacterium substantially equivalent" indicates a microorganism belonging to the genus *Clostridium bolteae* and capable of exhibiting an effect according to the present aspect, such as expressing the enzyme according to the present aspect, further having a base sequence of the 16S rRNA gene with a homology of preferably 98% or higher, more preferably 99% or higher, and even more preferably 100% to the base sequence of the 16S rRNA gene of the deposited strain, and preferably having the same mycological properties as those of the deposited strain. In addition, as long as the effect of the present aspect is not impaired, the strain may be a strain bred from the deposited strain or a strain substantially equivalent to the deposited strain by mutation treatment, genetic recombination, selection of a natural mutant strain, or the like.

This also applies to the other strains described above.

The microorganism belonging to the genus *Clostridium* is cultured in a common medium used in culturing anaerobes, such as Anaerobe Basal Broth (CM0957, available from ThermoFisher Scientific), Wilkins-Chalgren Anaerobe Broth (CM0643, available from ThermoFisher Scientific), GAM medium (available from Nissui Pharmaceutical Co., Ltd.), or a modified GAM medium (available from Nissui Pharmaceutical Co., Ltd.).

The culturing temperature is preferably 25°C or higher, more preferably 30°C or higher, and even more preferably 33°C or higher, and is also preferably 45°C or lower, more preferably 42°C or lower, and even more preferably 40°C or lower.

Furthermore, a water-soluble organic material can be added as a carbon source, for example. The water-soluble organic material is, for example, a saccharide, such as sorbose, fructose, glucose, dextrin, or a soluble starch; an alcohol, such as methanol; an organic acid, such as valeric acid, butyric acid, propionic acid, acetic acid, formic acid, or succinic acid; or an amino acid, such as arginine, methionine, phenylalanine, valine, or glutamic acid.

The concentration of organic material added as a carbon source in the culture medium can be adjusted, as appropriate, to facilitate efficient growth. Typically, the concentration can be selected in a range of from 0.1 to 10 wt/vol%.

In addition to the carbon source described above, a nitrogen source may be added to the culture medium. Various nitrogen compounds that may be used ordinarily in cultivation or fermentation can be used as the nitrogen source. Preferred inorganic nitrogen sources are ammonium salts and nitrates. More preferable examples include ammonium sulfate, ammonium chloride, ammonium phosphate, ammonium hydrogen phosphate, triammonium citrate, potassium nitrate and sodium nitrate.

On the other hand, a preferred organic nitrogen source is an amino acid, a yeast extract, a peptone (such as a milk-derived peptone, a soybean-derived peptone, or a soybean-derived peptide), a meat extract (e.g., such as Lab Lemco powder, a bonito extract, a tuna extract, a bonito extract, a bouillon, or a shellfish extract), a liver extract, a digestive serum powder, or the like. A more preferred organic nitrogen source is arginine, cysteine, citrulline, lysine, tryptophan, a yeast extract, or a peptone.

Furthermore, in addition to the carbon source or nitrogen source, microbial growth factors such as extracts, vitamins, and metal salts and inorganic compounds can also be added to the culture medium. Examples of extracts include hemin, heme iron, digestive serum powder, liver extract, and blood digestion products. Examples of vitamins include biotin, folic acid, pyridoxal, thiamine, riboflavin, nicotinic acid, nicotinamide, pantothenic acid, vitamin B12, thioctic acid, p-aminobenzoic acid, and vitamin K. Examples of metal salts and inorganic compounds include potassium dihydrogen phosphate, magnesium sulfate, manganese sulfate, sodium chloride, cobalt chloride, calcium chloride, zinc sulfate, copper sulfate, alum, sodium molybdate, potassium chloride, boric acid, and the like, nickel chloride, sodium tungstate, sodium selenate, sodium selenite, ammonium iron(II) sulfate, iron(II) citrate, sodium acetate trihydrate, magnesium sulfate heptahydrate, and manganese sulfate tetrahydrate. These metals may also be added in the form of mineral yeast.

Methods for producing a culture solution by adding plant- and/or animalderived growth cofactors such as these inorganic compounds and vitamins are well known. The culture medium can be a liquid, a semi-solid, or a solid. A preferred form of the culture medium is a liquid culture medium.

Production of the enzyme according to the present aspect by the microorganism belonging to the genus *Clostridium* is induced by adding urolithins having a hydroxyl group at the 9-position, which are raw materials (substrates), to the medium. The raw material (substrate) is preferably added at an amount at which the concentration in the culture medium is from 0.01 g/L to 20 g/L.

To collect the enzyme according to the present aspect produced by the microorganism belonging to the genus *Clostridium,* the culture is collected after production of the enzyme according to the present aspect, and the microorganism is crushed in a buffer solution to which a reducing agent, such as cysteine, 2-mercaptoethanol, or dithiothreitol, and/or a protease inhibitor, such as phenylmethane sulfonyl fluoride (PMFS), pepstatin A, or ethylenediaminetetraacetic acid, has been added to obtain a cell-free extract, and the enzyme can be purified from the cell-free extract by appropriately combining a fractionation based on protein solubility, chromatography of various types, and/or the like. These may all be carried out according to routine methods.

One, two or three subunits that are included in the *Clostridium bolteae* JCM 12243 strain and presumed to constitute the enzyme according to the present aspect are preferably one or a combination of two or three selected from the following subunits: CbUroC1; CbUroC2; and CbUroC3.

The amino acid sequences of CbUroC1, CbUroC2, and CbUroC3 are sequences represented by SEQ ID NOS: 1 to 3, respectively.

The base sequences of the genes encoding CbUroC1, CbUroC2, and CbUroC3 are sequences represented by SEQ ID NOS: 10 to 12, respectively.

In addition, the base sequences of the genes encoding CbUroC1, CbUroC2, and CbUroC3 may be subjected to modification, such as optimization, for expression of the genes using a heterologous host (heterologous expression). For example, a modification, such as optimization, may be made for expression in a microorganism belonging to the genus *Rhodococcus.* The base sequences of the genes encoding CbUroC1, CbUroC2, and CbUroC3, the genes optimized for expression in the microorganism belonging to the genus *Rhodococcus,* are, for example, sequences represented by SEQ ID NOS: 19 to 21, respectively.

One, two or three subunits that are included in the *Clostridium asparagiforme* DSM 15981 strain and presumed to constitute the enzyme according to the present aspect are preferably one or a combination of two or three selected from the following subunits: CaUroC1; CaUroC2; and CaUroC3.

The amino acid sequences of CaUroC1, CaUroC2, and CaUroC3 are sequences represented by SEQ ID NOS: 4 to 6, respectively.

The base sequences of the genes encoding CaUroC1, CaUroC2, and CaUroC3 are sequences represented by SEQ ID NOS: 13 to 15, respectively.

One, two or three subunits that are included in the *Clostridium citroniae* DSM 19261 strain and presumed to constitute the enzyme according to the present aspect are preferably one or a combination of two or three selected from the following subunits: CcUroC1; CcUroC2; and CcUroC3.

The amino acid sequences of CcUroC1, CcUroC2, and CcUroC3 are sequences represented by SEQ ID NOS: 7 to 9, respectively.

The base sequences of the genes encoding CcUroC1, CcUroC2, and CcUroC3 are sequences represented by SEQ ID NOS: 16 to 18, respectively.

Thus, the enzyme according to the present aspect is preferably an enzyme containing:
one or more selected from the group consisting of an amino acid sequence represented by SEQ ID NO: 1, an amino acid sequence represented by SEQ ID NO: 2, and an amino acid sequence represented by SEQ ID NO: 3;
one or more selected from the group consisting of an amino acid sequence represented by SEQ ID NO: 4, an amino acid sequence represented by SEQ ID NO: 5, and an amino acid sequence represented by SEQ ID NO: 6; or
one or more selected from the group consisting of an amino acid sequence represented by SEQ ID NO: 7, an amino acid sequence represented by SEQ ID NO: 8, and an amino acid sequence represented by SEQ ID NO: 9.

In addition, as long as CbUroC1 has the activity of dehydroxylation of the hydroxyl group at the 9-position of urolithins, when only CbUroC1 itself serves as one subunit or when CbUroC1 constitutes a unit together with the other subunits, which are CbUroC2 and/or CbUroC3, CbUroC1 may be a protein composed of amino acids where one to a plurality of amino acids is substituted or deleted, or one to a plurality of amino acids is inserted or added in the amino acid sequence represented by SEQ ID NO: 1. The expression "one to a plurality of" means preferably from 1 to 29, more preferably from 1 to 28, even more preferably from 1 to 20, and particularly preferably from 1 to 10, and the same is true when an amino acid is added to the N-terminal side and/or the C-terminal side. This also applies to CaUroC1 and CcUroC1.

The substitution is preferably a conservative substitution. A conservative substitution is a mutual substitution between Phe, Trp, and Tyr when the substitution site is an aromatic amino acid, between Leu, Ile, and Val when the substitution site is a hydrophobic amino acid, between Gin and Asn when the substitution site is a polar amino acid, between Lys, Arg, and His when the substitution site is a basic amino acid, between Asp and Glu when the substitution site is an acidic amino acid, or between Ser and Thr when the substitution site is an amino acid having a hydroxyl group. Examples of the conservative substitution specifically include a substitution of Ala with Ser or Thr, a substitution of Arg with Gln, His, or Lys, a substitution of Asn with Glu, Gln, Lys, His, or Asp, a substitution of Asp with Asn, Glu, or Gln, a substitution of Cys with Ser or Ala, a substitution of Gin with Asn, Glu, Lys, His, Asp, or Arg, a substitution of Glu with Gly, Asn, Gln, Lys, or Asp, a substitution of Gly with Pro, a substitution of His with Asn, Lys, Gln, Arg, or Tyr, a substitution of Ile with Leu, Met, Val, or Phe, a substitution of Leu with Ile, Met, Val, or Phe, a substitution of Lys with Asn, Glu, Gln, His, or Arg, a substitution of Met with Ile, Leu, Val, or Phe, a substitution of Phe with Trp, Tyr, Met, Ile, or Leu, a substitution of Ser with Thr or Ala, a substitution of Thr with Ser or Ala, a substitution of Trp with Phe or Tyr, a substitution of Tyr with His, Phe, or Trp, and a substitution of Val with Met, Ile, or Leu. This also applies to CaUroC1 and CcUroC1.

In addition, as long as CbUroC1 has the activity of dehydroxylation of the hydroxyl group at the 9-position of urolithins, when only CbUroC1 itself serves as one subunit or when CbUroC1 constitutes a unit together with the other subunits, which are CbUroC2 and/or CbUroC3, CbUroC1 may be a protein having a homology of 80% or higher, preferably of 90% or higher, more preferably of 95% or higher, even more preferably 97% or higher, and particularly preferably 99% or higher to the full length of the amino acid sequence represented by SEQ ID NO: 1. This also applies to CaUroC1 and CcUroC1.

In addition, as long as CbUroC1 has the activity of dehydroxylation of the hydroxyl group at the 9-position of urolithins, when only CbUroC1 itself serves as one subunit or when CbUroC1 constitutes a unit together with the other subunits, which are CbUroC2 and/or CbUroC3, CbUroC1 may be a protein encoded by a polynucleotide that hybridizes with the base sequence represented by SEQ ID NO: 10 under stringent conditions. "Stringent conditions" include, for example, conditions in which polynucleotides having homology of, for example, 80% or greater, preferably 90% or greater, more preferably 95% or greater, even more preferably 97% or greater, and particularly preferably 99% or greater hybridize, while polynucleotides having homology less than this do not hybridize. This also applies to CaUroC1 and CcUroC1.

The amino acid substitution, the protein homology, the stringent conditions, and the like also apply to CbUroC2. However, for CbUroC2, the expression "one to a plurality of" described above is preferably from 1 to 17, more preferably from 1 to 16, even more preferably from 1 to 10, still more preferably from 1 to 5, and particularly preferably from 1 to 3. This also applies to CaUroC2 and CcUroC2.

In addition, the amino acid substitution, the protein homology, the stringent conditions, and the like also apply to CbUroC3. However, for CbUroC3, the expression "one to a plurality of" described above is preferably from 1 to 79, more preferably from 1 to 78, even more preferably from 1 to 50, still more preferably from 1 to 30, and particularly preferably from 1 to 10. This also applies to CaUroC3 and CcUroC3.

The activity of dehydroxylation of the hydroxyl group at the 9-position of urolithins by the enzyme according to the present aspect can be evaluated, for example, as in Experimental Example 5 described later.

### <2. Polynucleotide>

Another aspect of the present disclosure is a polynucleotide encoding the enzyme according to the above aspect.

A specific example is:
a polynucleotide containing one or more selected from the group consisting of the base sequence represented by SEQ ID NO: 10, the base sequence represented by SEQ ID NO: 11, and the base sequence represented by SEQ ID NO: 12;
a polynucleotide containing one or more selected from the group consisting of the base sequence represented by SEQ ID NO: 13, the base sequence represented by SEQ ID NO: 14, and the base sequence represented by SEQ ID NO: 15; or
a polynucleotide containing one or more selected from the group consisting of the base sequence represented by SEQ ID NO: 16, the base sequence represented by SEQ ID NO: 17, and the base sequence represented by SEQ ID NO: 18.

The details of each base sequence are as described in the previous section "1. Enzyme dehydroxylating hydroxyl group at 9-position of urolithins".

In addition, as long as the base sequence of the gene encoding GuUroC1 has the activity of dehydroxylation of the hydroxyl group at the 9-position of urolithins, when only GbUroC1 itself serves as one subunit or when GbUroC1 constitutes a unit together with the other subunits, which are GbUroC2 and/or GbUroC3, the base sequence of the gene encoding GbUroC1 may be a base sequence that hybridizes with the base sequence represented by SEQ ID NO: 10 under stringent conditions. "Stringent conditions" include, for example, conditions in which polynucleotides having homology of, for example, 80% or greater, preferably 90% or greater, more preferably 95% or greater, even more preferably 97% or greater, and particularly preferably 99% or greater hybridize, while polynucleotides having homology less than this do not hybridize.

This also applies to the base sequence of the gene encoding GbUroC2 and the base sequence of the gene encoding GbUroC3. In addition, this also applies to the base sequence of the gene encoding GaUroC1, the base sequence of the gene encoding GaUroC2, and the base sequence of the gene encoding GaUroC3. Furthermore, this also applies to the base sequence of the gene encoding GcUroC1, the base sequence of the gene encoding GcUroC2, and the base sequence of the gene encoding GcUroC3.

### <3. Genetic engineering aspects>

Other aspects of the present disclosure include a recombinant vector containing the polynucleotide, a transformant in which the polynucleotide is expressively retained, or in which the vector is expressively retained, and a method for producing a protein encoded by the polynucleotide, the method including a step of culturing the transformant. The protein is the "enzyme that dehydroxylates the hydroxyl group at the 9-position of urolithins".

An expression vector that expresses the enzyme can be formed by inserting the polynucleotide into a known expression vector. The transformant can be obtained by transforming microorganisms or the like using the expression vector, and the transformant can then be cultured, etc. to produce the enzyme, and thereby the enzyme can be obtained.

Examples of host vector systems include lactic acid bacteria, such as *Lactococcus lactis subsp. Cremoris-vector-pNZ8 148* (available from MoBiTech, Inc.), *Lactococcus lactis-*pGKV11 (Appl. Environ. Microbiol., 50, 540-542 (1985)), and *Lactococcus lactislStreptococcus thermophiluslStreptococcus faecalis,* and the like - pBE194 (JP 06-253861 A), and *Lactococcus lactis subsp. lactis* / *Lactobacillus delbrueckii,* and the like - pSYE1 (JP 05-176776 A); and bifidobacteria, such as *Bifidobacterium adolescentis-*pKKT427 (Nucleic Acids Research, 2009, Vol. 37, No. 1 e3 doi: 10. 1093/nar/gkn884), a wide-range of *Lactobacillus bifidus-pNC7,* such as *Bifidobacterium longum* (Res. Microbiol., 147, 133-143 (1996)), and *Bifidobacterium* longum-pBS423 (Appl. Environ. Microbiol., 78, 4984-4994 (2012).

Examples of anaerobic bacteria include *Clostridium acetobutylicum -* pTY 10 (Agric. Biol. Chem., 54 (2), 437-441 (1990))/ pMTL500E (Microbiol. SCI. 5: 310-315 (1988)), and ACE vector (Anaerobe, 41, 104-112 (2016)).

While expression of the enzyme requires an anaerobic environment, heterologous expression of the gene does not necessarily require an anaerobic host-vector system. For example, the *Rhodococcus erythropolis* -pTip, pNit, pCpi Vector Series (Hokkaido System Science Co., Ltd., Biotechnol. Bioeng., 86, 136-148 (2004)) can be suitably used. A specific example of the host includes the L88 strain, and a specific example of the vector includes pTipQC1. In addition, an *Escherichia coli-*pET vector can be suitably used. Specific examples of the host include the Rosetta2 (DE3) strain, and specific examples of the vector include pET21b.

In addition to microorganisms, various hosts and vector systems have been developed with respect to plants and animals. For example, a system in which silkworms are used (Nature 315, 592-594 (1985)), and a system that expresses heterologous proteins in large amounts in plants such as rapeseed, corn, and potatoes have been developed, and these systems may be used.

### <4. Method for dehydroxylating hydroxyl group at 9-position of urolithins >

Another aspect of the present disclosure is a method for dehydroxylating the hydroxyl group at the 9-position of urolithins. Specifically, the method includes dehydroxylating a hydroxyl group at the 9-position of urolithins having a hydroxyl group at the 9-position.

The present aspect is a method for dehydroxylating a hydroxyl group at the 9-position of urolithins, the method including step (I) below:
(I) bringing one or more selected from (i) to (iv) below into contact with urolithins having a hydroxyl group at the 9-position to dehydroxylate the hydroxyl group at the 9-position:
   (i) the enzyme described in the section "1. Enzyme dehydroxylating hydroxyl group at 9-position of urolithins";
   (ii) a protein encoded by the polynucleotide described in the section "2. Polynucleotide";
   (iii) a microorganism producing the enzyme described in (i) above or the protein described in (ii) above; and
   (iv) a treated product of the microorganism described in (iii) above.

### (Urolithins)

The details of the urolithins in the present aspect are as described in the previous section "1. Enzyme dehydroxylating hydroxyl group at 9-position of urolithins".

### (Step (I))

In step (I), (i) the enzyme and (ii) a protein encoded by the polynucleotide are each brought into contact with urolithins having a hydroxyl group at the 9-position to dehydroxylate the hydroxyl group present at the 9-position in the urolithins. In addition, for (iii) a microorganism producing the enzyme described in (i) above or the protein described in (ii) above, and (iv) a treated product of the microorganism described in (iii) above, the enzyme or the protein contained in a microorganism or in a treated product of the microorganism is brought into contact with urolithins having a hydroxyl group at the 9-position to dehydroxylate the hydroxyl group present at the 9-position in the urolithins.

With respect to (iii) the microorganisms that produce the enzyme described in (i) above or the protein described in (ii) above, and (iv) the treated product of the microorganisms described in (iii) above, the microorganisms may be obtained through a genetic engineering technique.

The microorganism can be exemplified by microorganisms belonging to the genus *Clostridium.* Preferred specific examples of the microorganism belonging to the genus *Clostridium* include the same microorganisms as those described in the section "1. Enzyme dehydroxylating hydroxyl group at 9-position of urolithins".

The (i) enzyme and the (ii) protein encoded by a polynucleotide are not limited those that have been purified, and may also include those that have been partially purified.

Furthermore, examples of (iv) the treated product of the microorganisms include the microorganisms in which the permeability of the cell membrane has been changed by treatment using a surfactant or an organic solvent such as toluene, a cell-free extract in which bacterial cells have been crushed by a treatment using glass beads or enzymes, or a treated product obtained by partially purifying the same.

For a case in which one or more components selected from the aforementioned (i) to (iv) are to be brought into contact with urolithins having a hydroxyl group at the 9-position, such contact can be implemented in water; in an organic solvent that does not easily dissolve in water, for example, in an organic solvent such as ethyl acetate, butyl acetate, toluene, chloroform, and n-hexane; or in a two-phase mixed system of the organic solvent and an aqueous medium such as ethanol or acetone. The contact can also be implemented by immobilizing the one or more components selected from the aforementioned (i) to (iv), or the contact can be implemented using a membrane reactor or the like.

Furthermore, if the one or more components selected from (i) to (iv) are the microorganisms that produce the enzyme described in (i) above or the protein described in (ii) above, the contact can be implemented in a culture medium or a solution normally used in culturing the microorganisms, such as a phosphate buffer.

The temperature in Step (I) is preferably 25°C or higher, more preferably 30°C or higher, and even more preferably 33°C or higher, and is also preferably 55°C or lower, more preferably 50°C or lower, and even more preferably 42°C or lower.

The pH in Step (I) is preferably 4.0 or higher, more preferably 5.0 or higher, and even more preferably 5.5 or higher, and is also preferably 9.0 or less, more preferably 8.0 or less, and even more preferably 7.0 or less.

In Step (I), the concentration in the reaction solution of urolithins having a hydroxyl group at the 9-position, which is a raw material (substrate) in step (I), is 0.001 g/L or greater, preferably 0.01 g/L or greater, and more preferably 0.1 g/L or greater, and is also not greater than 100 g/L, preferably not greater than 20 g/L, and more preferably not greater than 10 g/L.

The present aspect may include a step of quantitatively determining the resulting product (quantitative determination step). The quantification method can be in accordance with routine methods. For example, ethyl acetate to which an acid such as formic acid has been added as necessary, is added to the culturing solution and stirred well, after which the mixture is centrifuged, and the ethyl acetate layer is extracted. The same operation can be implemented several times as necessary, and the extracted ethyl acetate layers can be combined to obtain a liquid extract of the product. The liquid extract is concentrated under reduced pressure using an evaporator or the like, dried, and dissolved in methanol. As an example, the obtained solution is then filtered using a membrane such as a polytetrafluoroethylene (PTFE) membrane to remove insoluble matter, and the resulting product can then be quantified using high performance liquid chromatography.

The present aspect may also include a step of recovering the resulting product. The recovery step includes steps such as a purification step and a concentration step. As a purification treatment in the purification step, treatments such as sterilization of the microorganism through heat; disinfection through a method such as microfiltration (MF) or ultrafiltration (UF); removal of solids and polymeric substances; extraction using an organic solvent, an ionic liquid, or the like; and adsorption and decolorization using a hydrophobic adsorbent, an ion exchange resin, an activated carbon column, or the like can be implemented. Furthermore, examples of a concentration treatment in the concentration step include concentration using an evaporator, reverse osmosis membrane, or the like.

In addition, a solution containing the resulting product can be formed into a powder through freeze drying, spray drying, or the like. In the formation of a powder, an excipient such as lactose, dextrin, or corn starch can be added.

### <5. Method for producing urolithin A>

Another aspect of the present disclosure is a method (first aspect) for producing urolithin A.

The present aspect is a method for producing urolithin A, the method including steps (I) and (II) below:
(I) allowing a microorganism having an ability to produce urolithin C from ellagic acid to produce urolithin C from ellagic acid; and
(II) bringing one or more selected from (i) to (iv) below into contact with the urolithin C to produce urolithin A:
   (i) the enzyme described in the section "1. Enzyme dehydroxylating hydroxyl group at 9-position of urolithins";
   (ii) a protein encoded by the polynucleotide described in the section "2. Polynucleotide";
   (iii) a microorganism producing the enzyme described in (i) above or the protein described in (ii) above; and
   (iv) a treated product of the microorganism described in (iii) above.

### (Step (I)

In step (I), a microorganism having an ability to produce urolithin C from ellagic acid is allowed to produce urolithin C from ellagic acid.

Examples of the microorganism having an ability to produce urolithin C from ellagic acid include microorganisms belonging to the genus *Gordonibacter,* and microorganisms belonging to the genus *Eggerthella.*

Among the microorganisms belonging to the genus *Gordonibacter,* the microorganism is preferably a microorganism belonging to the genus *Gordonibacter pamelaeae,* a microorganism belonging to the genus *Gordonibacter urolithinfaciens,* and/or a microorganism belonging to the genus *Gordonibacter faecihominis.*

Furthermore, the microorganism belonging to *Gordonibacter pamelaeae* is more preferably the DSM 19378 strain, and the microorganism belonging to *Gordonibacter urolithinfaciens* is more preferably DSM 27213 strain.

Among the microorganism belonging to the genus *Eggerthella,* the microorganism is preferably *Eggerthella sp.* DC 3563 (NITE BP-02376) strain.

One or two or more species of the microorganisms described above may be used regardless of the genus, species, and strain.

The strain to which accession No. of *Eggerthella sp.* DC 3563 (NITE BP-02376) was assigned was internationally deposited with Patent Microorganisms Depositary, National Institute of Technology and Evaluation (NITE) (#122, 2-5-8 Kazusa Kamatari, Kisarazu-shi, Chiba-ken, 292-0818) as of November 11, 2016, under the Budapest Treaty.

Examples of the method for allowing a microorganism having an ability to produce urolithin C from ellagic acid to produce urolithin C from ellagic acid include a common method. In addition, urolithin C may be produced by a method similar to the method for culturing a microorganism belonging to the genus *Clostridium* described in the section "1. Enzyme dehydroxylating hydroxyl group at 9-position of urolithins".

### (Step (II))

In step (II), one or more selected from (i) to (iv) below are brought into contact with urolithin C to produce urolithin A:
(i) the enzyme described in the section "1. Enzyme dehydroxylating hydroxyl group at 9-position of urolithins";
(ii) a protein encoded by the polynucleotide described in the section "2. Polynucleotide";
(iii) a microorganism producing the enzyme described in (i) above or the protein described in (ii) above; and
(iv) a treated product of the microorganism described in (iii) above.

In step (II), (i) the enzyme and (ii) a protein encoded by the polynucleotide are each brought into contact with urolithin C having a hydroxyl group at the 9-position to dehydroxylate the hydroxyl group present at the 9-position in the urolithin C. In addition, for (iii) a microorganism producing the enzyme described in (i) above or the protein described in (ii) above, and (iv) a treated product of the microorganism described in (iii) above, the enzyme or the protein contained in a microorganism or in a treated product of the microorganism is brought into contact with urolithin C having a hydroxyl group at the 9-position to dehydroxylate the hydroxyl group present at the 9-position in the urolithin C.

The details of the aspect are similar to the details described in the previous section "4. Method for dehydroxylating hydroxyl group at 9-position of urolithins".

Steps (I) and (II) may or may not be implemented in the same system.

The above "implemented in the same system" means that the series in which the urolithin C produced in Step (I) is used as is as the urolithin C in Step (II) to produce the urolithin A in Step (II) is continuously implemented in the same system. That is, for example, this series does not include a step of separating and/or purifying the urolithin C produced in Step (I). Note that when microorganisms are used in each step, the microorganism in each step may be the same or different.

The present aspect may include other steps. For example, the present aspect may include quantifying urolithin A (quantification), purifying urolithin A (purification), and/or concentrating urolithin A (concentration). The details of those are similar to the details described in the previous section "4. Method for dehydroxylating hydroxyl group at 9-position of urolithins". Furthermore, the present aspect may include forming a solution containing urolithin A into a powder by freeze-drying, spray drying, or the like. In the formation of a powder, a filler, such as lactose, dextrin, or corn starch, can be added.

### <6. Method for producing urolithin B>

Another aspect of the present disclosure is a method for producing urolithin B.

The present aspect is a method for producing urolithin B, the method including steps (I) to (III) below:
(I) allowing a microorganism having an ability to produce urolithin C from ellagic acid to produce urolithin C from ellagic acid;
(II) allowing a microorganism having an ability to produce isourolithin A from urolithin C to produce isourolithin A from the urolithin C; and
(III) bringing one or more selected from (i) to (iv) below into contact with the isourolithin A to produce urolithin B:
   (i) the enzyme described in the section "1. Enzyme dehydroxylating hydroxyl group at 9-position of urolithins";
   (ii) a protein encoded by the polynucleotide described in the section "2. Polynucleotide";
   (iii) a microorganism producing the enzyme described in (i) above or the protein described in (ii) above; and
   (iv) a treated product of the microorganism described in (iii) above.

### (Step (I))

For step (I), the content described in the previous "5. Method for producing urolithin A" is incorporated by reference.

### (Step (II))

In step (II), a microorganism having an ability to produce isourolithin A from urolithin C is allowed to produce isourolithin A from the urolithin C.

Examples of the microorganism having an ability to produce isourolithin A from urolithin C include microorganisms belonging to the genus *Slackia.*

Among the microorganisms belonging to the genus *Slackia,* the microorganism is preferably a microorganism belonging to *Slackia heliotrinireducens.*

Furthermore, among the microorganisms belonging to *Slackia heliotrinireducens,* the microorganism is more preferably *Slackia heliotrinireducens* DSM 20476 strain.

In step (III), one or more selected from (i) to (iv) below are brought into contact with isourolithin A to produce urolithin B:
(i) the enzyme described in the section "1. Enzyme dehydroxylating hydroxyl group at 9-position of urolithins";
(ii) a protein encoded by the polynucleotide described in the section "2. Polynucleotide";
(iii) a microorganism producing the enzyme described in (i) above or the protein described in (ii) above; and
(iv) a treated product of the microorganism described in (iii) above.

In step (III), (i) the enzyme and (ii) a protein encoded by the polynucleotide are each brought into contact with isourolithin A having a hydroxyl group at the 9-position to dehydroxylate the hydroxyl group present at the 9-position in the isourolithin A. In addition, for (iii) a microorganism producing the enzyme described in (i) above or the protein described in (ii) above, and (iv) a treated product of the microorganism described in (iii) above, the enzyme or the protein contained in a microorganism or in a treated product of the microorganism is brought into contact with isourolithin A having a hydroxyl group at the 9-position to dehydroxylate the hydroxyl group present at the 9-position in the isourolithin A.

The details of the aspect are similar to the details described in the previous section "4. Method for dehydroxylating hydroxyl group at 9-position of urolithins".

Steps (I) to (III) may or may not be implemented in the same system.

The phrase "implemented in the same system" described above refers to continuously implementing a series of the following processes in the same system: urolithin C produced in step (I) is used as is as urolithin C in step (II) to produce isourolithin A in the step (II); and the isourolithin A is used as is as isourolithin A in step (III) to produce urolithin B in the step (III). That is, for example, this series does not include a step of separating and/or purifying the urolithin C produced in Step (I). Note that when microorganisms are used in each step, the microorganism in each step may be the same or different.

The present aspect may include other steps. The details are similar to the details described in the previous section "5. Method for producing urolithin A".

### EXAMPLES

Examples are described below, but none of the examples shall be construed as limiting.

Experimental Example 1: preparation of bacterial cells for proteome analysis

The *Clostridium bolteae* JCM 12243 strain was inoculated into 10 mL of an ABB medium (ANAEROBE BASAL BROTH, Oxoid Limited) containing 0.1% of urolithin C or an ABB medium not containing urolithin C and cultured at 37°C for 48 hours under an anaerobic gas (N₂/H₂/CO₂ = 80:10:10) environment.

The culture solution was centrifuged to collect bacterial cells, and the bacterial cells were used for proteome analysis.

### Experimental Example 2: proteome analysis

The bacterial cells prepared in Experimental Example 1 were lysed using a lysate (7 M urea, 2 M thiourea, 2% 3-[3-cholamidopropyl) dimethylammonio]propanesulfonate (CHAPS), 10 mM dithiothreitol (DTT), and a 50 mM tris-hydrochloric acid buffer (pH 7.0)), and subjected to proteome analysis by the method described in AMB Express, 2: 37 (2012).

As a result, proteins with significantly increased expression level, that is, proteins that had been induced to express by urolithin C, were detected when the bacterial cells were cultured in the medium containing urolithin C, compared with when the bacterial cells were cultured in the medium not containing urolithin C. EDP14314, EDP14313, and EDP14312 were identified by analysis based on genome analysis information. GENETYX (Genetyx Corporation) was used for the analysis of gene information, such as homology analysis.

### Experimental Example 3: protein function prediction

The functions of the amino acid sequences of EDP14314, EDP14313, and EDP14312 were predicted by a homology search with known proteins. The results are as follows.

EDP14314 is presumed to be "carbon monoxide dehydrogenase".

EDP14313 is presumed to be "a protein having an iron-sulfur (Fe-S) cluster".

EDP14312 is presumed to be "an enzyme catalyzing a redox reaction between hydroxybenzoyl-CoA and ferredoxin".

These EDP14314, EDP14313, and EDP14312 are presumably likely to be subunits constituting the enzyme dehydroxylating the hydroxyl group at the 9-position of urolithins. These genes were named CburoC1, CburoC2, and CburoC3. These are genes encoding CbUroC1, CbUroC2, and CbUroC3, respectively.

### Experimental Example 4: purification of enzyme (Culture of microorganism)

As a preculture, 10 mL of a GAM medium (Nihon Pharmaceutical Co., Ltd.) containing 0.1 mg/mL of urolithin C was added to a screw-top test tube (φ16.5 x 110 mm) and inoculated with the *Clostridium bolteae* JCM 12243 strain, and then shaken and cultured at 80 rpm in an anaerobic chamber at 37°C for 48 hours.

The preculture solution was inoculated into a 2-L Erlenmeyer flask containing 2.0 L of the same medium and then shaken and cultured at 120 rpm in an anaerobic chamber at 37°C for 48 hours.

The resulting culture solution was centrifuged (8,000 rpm, 10 minutes, 4°C), and bacterial cells were prepared as a precipitated fraction.

### (Preparation of ultracentrifuged supernatant)

The resulting bacterial cells were suspended in a 20 mM potassium phosphate buffer (KPB, pH 6.5) containing 1 mM DTT, and the bacteria were crushed using a KUBOTA Insonator Model 201M (KUBOTA Corporation). The liquid containing ultrasonically crushed bacteria cells was centrifuged at 20,000 x g at 4°C for 60 minutes, and a supernatant was obtained as a cell-free extract. The resulting cell-free extract was ultracentrifuged at 100,000 x g at 4°C for 90 minutes, and its supernatant fraction was obtained.

### (Purification of enzyme (ammonium sulphate fractionation))

Ammonium sulfate was added to the resulting supernatant fraction to give a final concentration of 30%. The mixture was stirred in an anaerobic chamber for one hour and then centrifuged at 20,000 x g at 4°C for 10 minutes, and its supernatant fraction (ammonium sulfate fraction (i)) was obtained. Ammonium sulfate was added to the ammonium sulphate fractionation (i) to give a final concentration of 50%. The mixture was stirred in an anaerobic chamber for one hour and then centrifuged at 20,000 x g at 4°C for 10 minutes, and its precipitated fraction was suspended in a 20 mM KPB (pH 6.5). This solution was desalted using PD-10D Desaultinf Columns containing 8.3 ml of Sephadex (trade name) G-25 Medium (GE Healthcare). After applying 3 mL of the sample, an operation of washing and desalting with 5 mL of a 20 mM KPB (pH 6.5) was repeated, the solution after desalting was concentrated, and an ammonium sulfate fraction (ii) was obtained.

### (Purification of enzyme (anion exchange chromatography))

The resulting ammonium sulfate fraction (ii) was subjected to anion exchange chromatography using a Hiprep DEAE 16 10 ff column.
Eluent A: 20 mM KPB (pH 6.5) containing 1 mM DTT
Eluent B: 20 mM KPB (pH 6.5) containing 1 mM DTT; and 1 M NaCl
Flow rate: 3 mL/min

Gradient elution from the eluate A to the eluate B was carried out, and an active fraction of the enzyme dehydroxylating the hydroxyl group at the 9-position of urolithins was collected as a DEAE fraction.

### (Purification of enzyme (hydrophobic column))

The resulting DEAE fraction was fractionated using a 5 mL of HiTrap TM Butyl Sepharose HP.
Eluent A: 20 mM KPB (pH 6.5) containing 1 mM DTT; and (NH₄)₂SO₄
Eluent B: 20 mM KPB (pH 6.5) containing 1 mM DTT
Flow rate: 3.0 mL/min

Gradient elution from the eluate A to the eluate B was carried out, and an active fraction of the enzyme dehydroxylating the hydroxyl group at the 9-position of urolithins was collected as a Butyl fraction.

### (Enzyme purification (gel filtration))

The resulting Butyl fraction was subjected to gel filtration chromatography using a Super 200 Increase 10/300.
Eluent A: 20 mM KPB (pH 6.5) containing 1 mM DTT; and 150 mM NaCl
Flow rate: 1.0 mL/min

An active fraction of the enzyme dehydroxylating the hydroxyl group at the 9-position of urolithins was collected as an Increase fraction.

### Experimental Example 5: measurement of enzyme activity

The activity of dehydroxylation of the hydroxyl group at the 9-position of urolithins was measured for each fraction obtained in Experimental Example 4. Urolithin C was used as the urolithins having a hydroxyl group at the 9-position. Dehydroxylation of the hydroxyl group at the 9-position produces urolithin A.

A 100 mM Gly/NaOH buffer (pH 9.5) containing 0.05 mg/mL of urolithin C, 5 mM of NADH, and 1 mM of DTT was mixed with a reaction solution for measuring the enzyme activity of each fraction corresponding to 40 mL of the medium to give a total volume of 500 µL, and the mixture was placed in a 1.5-mL tube. A gas phase in an anaerobic chamber was used as a gas phase (Vinyl Anaerobic Chamber available from Coy Labs), and the mixture was reacted at 45°C for 13 to 18 hours. After completion of the reaction, 200 µL of N,N-dimethylacetamide containing 10% formic acid was added and mixed. Then, the mixture was centrifuged (10,000 rpm, 4°C, 3 minutes), and the resulting supernatant was analyzed by HPLC.

The HPLC was performed under the following conditions, and the produced urolithin C was quantified (detection wavelength: 337 nm for urolithin C and 354 nm for urolithin A).

### <HPLC conditions>

Column: COSMOSIL Packed Column 5C18-AR II (4.6 mm in inner diameter x 150 mm in length, available from Nacalai Tesque, Inc.)
Eluent: ultrapure water (Milli Q water)/acetonitrile/formic acid = 80:20:1
Column temperature: 40°C
Flow rate: 1.0 mL/min
Detection: photodiode array (PDA)

One unit of the enzyme activity was defined as the amount of enzyme that catalyzes the production of 1 pmol of urolithin A per minute.

The results are shown in Table 1.

### [Table 1]

**Table 1**

| Step | Protein (mg) | Enzyme activity (U) | Specific activity (U/mg) | Purification ratio |
|---|---|---|---|---|
| Ultracentrifuged supernatant | 104.04 | 18.01 | 0.17 | 1 |
| Ammonium sulfate fraction | 22.26 | 12.05 | 0.54 | 3.13 |
| DEAE fraction | 7.36 | 8.02 | 1.09 | 6.29 |
| Butyl fraction | 0.33 | 0.80 | 2.47 | 14.25 |
| Increase fraction | 0.07 | 0.33 | 4.74 | 27.39 |

### Experimental Example 6 SDS-PAGE

The enzyme fractions purified thus far were subjected to sodium dodecyl sulfate-electrophoresis (SDS-PAGE) using a 5 to 20% gradient gel (e-PAGEL, E-R520L, available from ATTO Corp.), and the molecular weight was evaluated. A Protein Molecular Weight Marker (Broad) available from Takara Bio Inc. was used as the molecular weight marker.

The resulting bands were semi-dry blotted onto a PVDF membrane (Immobilom-P Transfer membrane, available from Millipore) using Porize Plot AE6677G (available from ATTO Corp.), each band was cut out, and the sequence was analyzed with an amino acid sequencer.

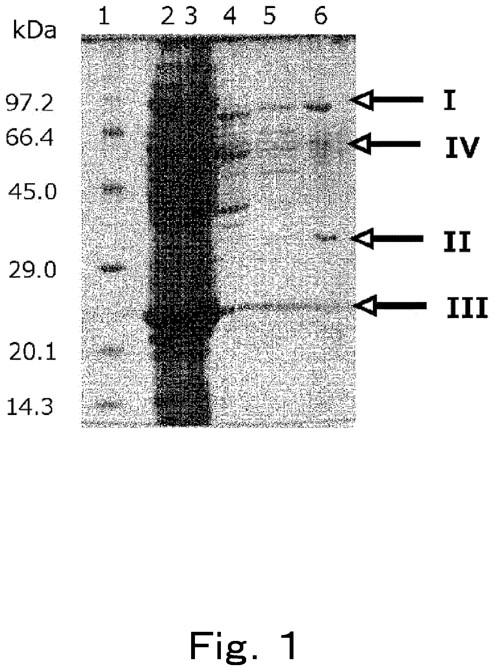

The results are shown in FIG. 1. In FIG. 1, 1 is a lane of molecular weight markers, 2 is a lane of the ultracentrifuged supernatant fraction, 3 is a lane of the ammonium sulphate fractionation (ii), 4 is a lane of the DEAE fraction, 5 is a lane of the Butyl fraction, and 6 is a lane of the Increase fraction.

As a result of analyzing the amino acid sequences of band I, band II, band III and band IV obtained in the Increase fraction in lane 6, four amino acid sequences were obtained. Band I was identified as CbUroC3, band II as CbUroC1, and band III as CbUroC2. The molecular weight of each band is estimated to be 86.7, 31.1, and 17.8 kDa, respectively. Band IV is presumed to be Shaperonin GroEL from its amino acid sequence.

### Experimental Example 7: effect of cofactor on enzyme activity

Solutions were prepared to contain a cofactor in a concentration as shown in Table 2 in the reaction solution for measuring the enzyme activity described in Experimental Example 5, and urolithin A produced after the reaction was quantified. The DEAE fraction was used as the enzyme.

The results are shown in Table 2. Increase in activity was confirmed when NADH or NADPH was individually added. No. 1 represents a control sample containing no cofactor but containing the enzyme. In addition, FAD refers to flavin adenine dinucleotide.

### [Table 2]

**Table 2**

| No. | Cofactor (mM) | | | | Relative activity |
|---|---|---|---|---|---|
| | NADH | NADPH | FAD | FMN | |
| 1 | - | - | - | - | 100% |
| 2 | 10 | - | - | - | 186% |
| 3 | - | 10 | - | - | 161 % |
| 4 | - | - | 0.2 | - | 95% |
| 5 | - | - | - | 0.2 | 96% |
| 6 | 10 | - | 0.2 | - | 102% |
| 7 | 10 | - | - | 0.2 | 130% |
| 8 | - | 10 | 0.2 | - | 72% |
| 9 | - | 10 | - | 0.2 | 134% |

### Experimental Example 8: temperature dependence of enzyme

The activity was measured in a solution containing 0.1 mg/mL of urolithin C, 5 mM of NADH, 1 mM of DTT, 100 mM Tris/HCl buffer (pH 8.0), and the DEAE fraction by changing the reaction temperature.

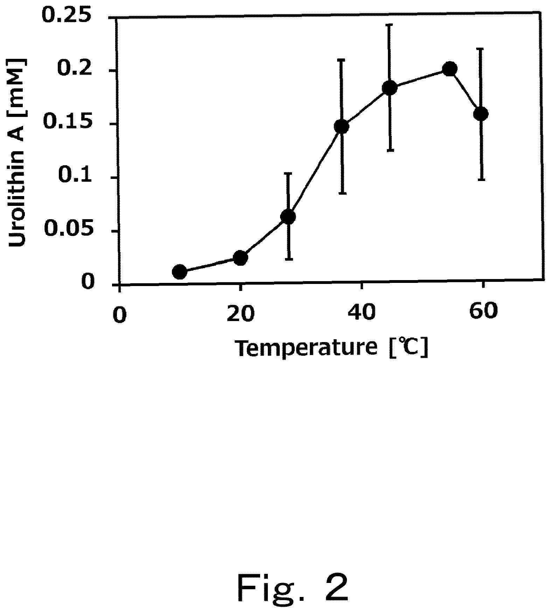

The results are shown in FIG. 2. The optimum temperature was 55°C, and the activity at that time was defined as 100%. In a range of 37 to 60°C, the enzyme exhibited an activity of 50% or higher.

### Experimental Example 9: pH dependence of enzyme

Under the activity measurement conditions described in Experimental Example 8, the activity was measured by changing the pH using the following buffers.
Citric acid buffer: 100 mM citric acid-sodium citrate buffer
Acetic acid buffer: 100 mM acetic acid-sodium acetate buffer
Potassium phosphate buffer: 100 mM potassium dihydrogen phosphatepotassium hydrogen phosphate buffer
Tris-HCl buffer: 100 mM Tris-hydrochloric acid buffer
Glycine buffer: 100 mM glycine-sodium hydroxide buffer
Carbonate buffer: 100 mM NaHCO₃-Na₂CO₃ buffer
Sodium phosphate buffer: 100 mM Na₂HPO₄-Na₃PO₄ buffer

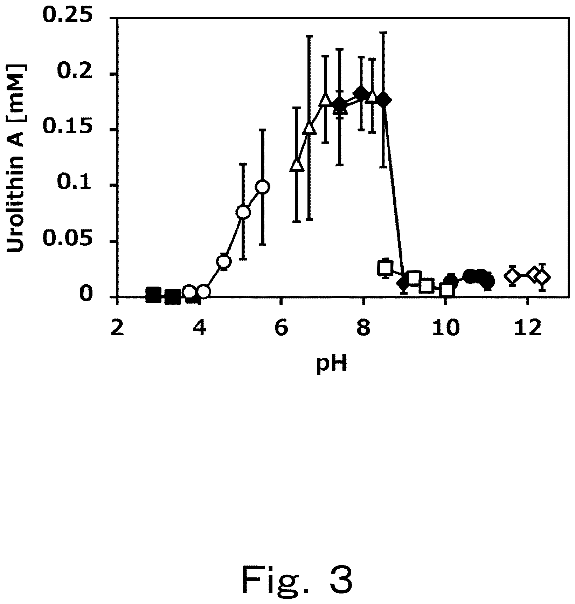

The results are shown in FIG. 3. The optimum pH was 8.0, and in a pH range of 6.0 to 8.5, the enzyme exhibited an activity of 60% or higher of that in the optimum conditions.

### Experimental Example 10: temperature stability of enzyme

The enzyme was maintained at 0 to 80°C for 24 hours, and then the activity was measured at the activity measurement conditions described in Experimental Example 8.

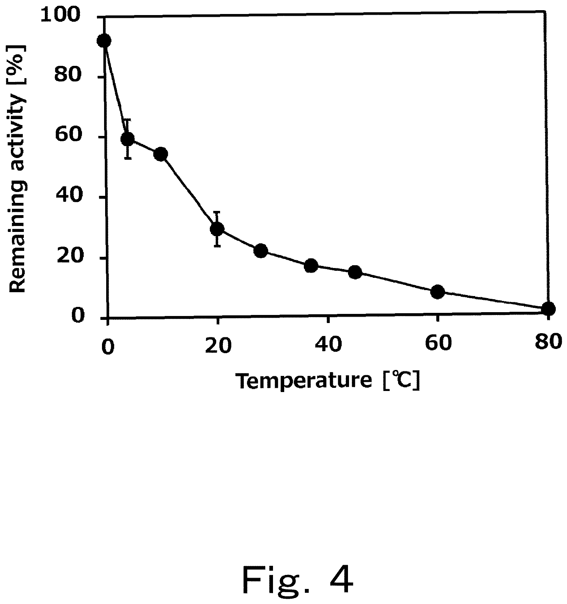

The results are shown in FIG. 4. The optimum temperature was 0°C, and the enzyme exhibited a residual activity of 60% or higher at 0 to 20°C when the enzyme activity before maintaining the enzyme for 24 hours was defined as 100%.

### Experimental Example 11: pH stability of enzyme

A volume of 1 mL of each of the following buffers (100 mM) was added to 56 µL of a purified enzyme solution (20 mM phosphate buffer, pH 6.5), and the mixture was maintained at 0°C for 24 hours. Each mixture was then concentrated by ultrafiltration, then 100 mM Tris/HCl buffer (pH 8.0) was added to restore the pH, and then the activity was measured under the activity measurement conditions described in Experimental Example 8.
Citric acid buffer: 100 mM citric acid-sodium citrate buffer
Acetic acid buffer: 100 mM acetic acid-sodium acetate buffer
Potassium phosphate buffer: 100 mM potassium dihydrogen phosphatepotassium hydrogen phosphate buffer
Tris-HCl buffer: 100 mM Tris-hydrochloric acid buffer
Glycine buffer: 100 mM glycine-sodium hydroxide buffer
Carbonate buffer: 100 mM NaHCO₃-Na₂CO₃ buffer
Sodium phosphate buffer: 100 mM Na₂HPO₄-Na₃PO₄ buffer

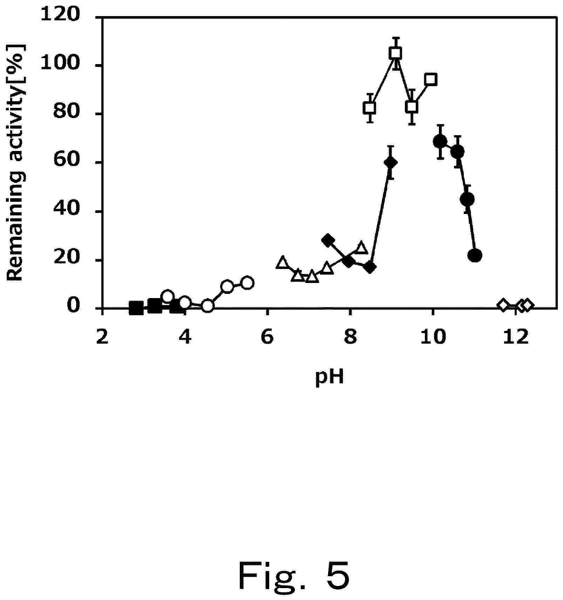

The results are shown in FIG. 5. The pH at which the enzyme was most stable was 8.5 (glycine buffer). The enzyme exhibited a residual activity of 80% or higher in a pH range of 8.0 to 10.0 when the enzyme activity before maintaining the enzyme for 24 hours was defined as 100%.

### Example 12: construction of vector expressing enzyme

Each of gene sequences of CbUroC1, CbUroC2, and CbUroC3, which are presumed to constitute the enzyme dehydrogenating a hydroxyl group at the 9-position of a urolithin derived from the *Clostridium bolteae* JCM 12243 strain, was optimized for heterologous expression using a microorganism belonging to the genus *Rhodococcus,* and base sequences represented by SEQ ID NOS: 19 to 21 were designed, respectively.

Each of base sequences represented by SEQ ID NO: 22 containing each of the base sequences represented by SEQ ID NOS: 19 to 21 was artificially synthesized and inserted using an In-Fusion HD Cloning Kit (available from Takara Bio Inc.) into a genus *Rhodococcus* expression vector pTipQC1 (Hokkaido System Science Co., Ltd.) cleaved with Nco I, and a plasmid for heterologous expression (pTipQC1_uroC123_opt.) was obtained.

Primers having the following base sequences were used in the In-Fusion reaction.

SYN6606 F01: 5’-TAAGAAGGAGATATACATGGTCCTCCCGCAGT
TCGA-3’ (SEQ ID NO: 23)

SYN6606 R02: 5'-GTGATGGTGGCCCATGTCACTTCGAGTTGCGG
TCGT-3' (SEQ ID NO: 24)

### Example 13: construction of transformant

The produced recombinant vector was introduced into *Rhodococcus erythropolis* L88 strain (Hokkaido System Science Co., Ltd.) by electroporation. Chloramphenicol was used as the selectable marker.

The formed colony was cultured (28°C) in an LB medium (20 µg/mL chloramphenicol), and a glycerol stock (30% glycerol) was produced from the culture solution, stored (-80°C), and used for expression of the enzyme later.

### Example 14: heterologous expression of enzyme

As a preculture, 5 mL of an LB medium containing 20 µg/mL of chloramphenicol was added to a 10-mL capacity test tube and inoculated with the transformed strain, and then shaken and cultured at 300 rpm at 28°C for 2 to 3 days.

A 1-L capacity Erlenmeyer flask containing 400 mL of an LB medium containing 20 µg/mL of chloramphenicol was inoculated with 1% of the preculture solution, and the medium was shaken and cultured at 120 rpm at 28°C for 9 hours. Then, 0.2 µg/mL of thiostrepton was added, and the enzyme was induced in an anaerobic environment for 48 hours.

Bacterial cells were collected by centrifugation (8,000 rpm, 20 minutes, 4°C) and washed twice with a physiological saline solution, and dormant bacterial cells were obtained.

### Example 15: preparation of cell-free extract

The washed bacterial cells were suspended in a 20 mM KPB (pH 6.5) containing 1 mM DTT and crushed using glass beads (2,700 rpm, 6 cycles of 60 sec on/60 sec off, 4°C).

The liquid containing crushed bacterial cells was centrifuged (20,000 g, 60 min, 4°C), and the supernatant was obtained as a cell-free extract.

### Example 16: SDS-PAGE

The cell-free extract (soluble fraction), the supernatant obtained by centrifugation of the liquid containing crushed bacterial cells described in Example 15, was subjected to sodium dodecyl sulphate-electrophoresis (SDS-PAGE) using a 5 to 20% gradient gel (e-PAGEL, E-R520L, available from ATTO Corp.), and the molecular weight was evaluated. A Protein Molecular Weight Marker (Broad) available from Takara Bio Inc. was used as the molecular weight marker.

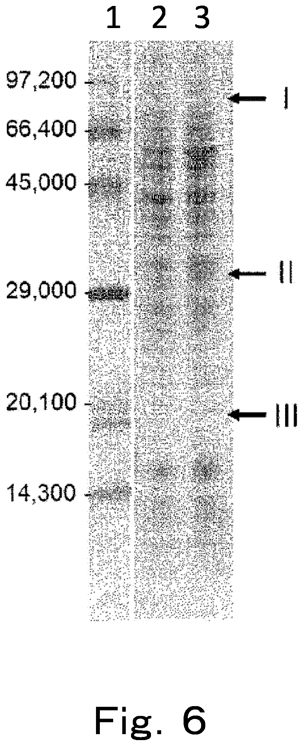

The results are shown in FIG. 6. In FIG. 6, 1 is a lane of molecular weight markers, 2 is a lane loaded with 1.0 µL of a cell-free extract (soluble fraction) prepared using pTipQC1 containing none of the genes encoding CbUroC1, CbUroC2, and CbUroC3, and 3 is a lane loaded with 1.0 µ L of the cell-free extract (soluble fraction) of Example 15 prepared using pTipQC1_uroC123_opt.

In lanes 2 and 3, CbUroC3 of band I, CbUroC1 of band II, and CbUroC2 of band III were confirmed. The molecular weight of each band was in good agreement with calculated values (86.7, 31.1, and 17.8 kDa, respectively).

### Example 17: measurement of activity of enzyme obtained by heterologous expression

The activity of dehydroxylation of the hydroxyl group at the 9-position of urolithins was measured. Urolithin C was used as the urolithins having a hydroxyl group at the 9-position. The enzyme activity was measured by quantifying urolithin A produced by dehydroxylation of the hydroxyl group at the 9-position.

A volume of 50 µL of a reaction solution for measuring the enzyme activity was placed in a 200-µL tube for PCR, the reaction solution containing: a 20 mM potassium phosphate buffer (KPB, pH 6.5) into which were added 0.5 mg/mL urolithin C, 5 mM reduced nicotinamide adenine diphosphate (NADH), and 1 mM dithiothreitol (DTT); and 15 µL of the cell-free extract obtained in Example 15. A gas phase in an anaerobic chamber was used as a gas phase (Vinyl Anaerobic Chamber available from Coy Labs), and the mixture was reacted at 45°C for 24 hours (200 rpm). After completion of the reaction, 100 µL of N,N-dimethylacetamide containing 1% formic acid was added and mixed. Then, the mixture was centrifuged (10,000 rpm, 4°C, 3 minutes), and the resulting supernatant was analyzed by HPLC.

The produced amount of urolithin A was 0 (not greater than the detection limit) in a control sample of the cell-free extract prepared using pTipQC1 containing none of the genes encoding CbUroC1, CbUroC2, and CbUroC3, whereas 4.33 mM of urolithin A was produced in a sample of the cell-free extract of Example 15 prepared using pTipQC1_uroC123_opt.

## Claims

1. An enzyme having properties (1) and (2):
(1) the enzyme dehydroxylates a hydroxyl group at a 9-position of urolithins; and
(2) the enzyme is activated by reduced nicotinamide adenine dinucleotide (NADH) and/or reduced nicotinamide adenine dinucleotide phosphate (NADPH).

2. The enzyme according to claim 1, having properties (3) to (5):
(3) an SDS-PAGE result includes a band indicating a relative molecular mass of 77,000 or greater and 95,000 or less;
(4) an optimum pH is 6.0 or higher and 8.5 or lower; and
(5) an optimum temperature is 37°C or higher and 60°C or lower.

3. The enzyme according to claim 1 or 2, derived from a microorganism belonging to the genus *Clostridium.*

4. The enzyme according to claim 3, wherein the microorganism belonging to the genus *Clostridium* is one or more selected from the group consisting of a microorganism belonging to *Clostridium bolteae,* a microorganism belonging to *Clostridium asparagiforme,* a microorganism belonging to *Clostridium citroniae,* and a microorganism belonging to *Clostridium sp.*

5. The enzyme according to claim 4, wherein
the microorganism belonging to *Clostridium bolteae* is one or more selected from the group consisting of *Clostridium bolteae* JCM 12243 strain, *Clostridium bolteae* DSM 29485 strain, and *Clostridium bolteae* DSM 15670 strain;
the microorganism belonging to *Clostridium asparagiforme* is *Clostridium asparagiforme* DSM 15981 strain;
the microorganism belonging to *Clostridium citroniae* is *Clostridium citroniae* DSM 19261 strain; and
the microorganism belonging to *Clostridium sp.* is *Clostridium sp.* DC3656 (NITE BP-02708) strain.

6. The enzyme according to any one of claims 1 to 5,
comprising an amino acid sequence represented by SEQ ID NO: 3;
comprising an amino acid sequence represented by SEQ ID NO: 6; or
comprising an amino acid sequence represented by SEQ ID NO: 9.

7. The enzyme according to any one of claims 1 to 6,
comprising an amino acid sequence represented by SEQ ID NO: 1, an amino acid sequence represented by SEQ ID NO: 2, and an amino acid sequence represented by SEQ ID NO: 3;
comprising an amino acid sequence represented by SEQ ID NO: 4, an amino acid sequence represented by SEQ ID NO: 5, and an amino acid sequence represented by SEQ ID NO: 6; or
comprising an amino acid sequence represented by SEQ ID NO: 7, an amino acid sequence represented by SEQ ID NO: 8, and an amino acid sequence represented by SEQ ID NO: 9.

8. A polynucleotide,
comprising a base sequence represented by SEQ ID NO: 12,
comprising a base sequence represented by SEQ ID NO: 15; or
comprising a base sequence represented by SEQ ID NO: 18.

9. A polynucleotide,
comprising a base sequence represented by SEQ ID NO: 10, a base sequence represented by SEQ ID NO: 11, and a base sequence represented by SEQ ID NO: 12;
comprising a base sequence represented by SEQ ID NO: 13, a base sequence represented by SEQ ID NO: 14, and a base sequence represented by SEQ ID NO: 15; or
comprising a base sequence represented by SEQ ID NO: 16, a base sequence represented by SEQ ID NO: 17, and a base sequence represented by SEQ ID NO: 18.

10. A recombinant vector comprising the polynucleotide according to claim 8 or 9.

11. A transformant, retaining the polynucleotide according to claim 8 or 9 in a manner that the polynucleotide can be expressed or retaining the vector according to claim 10 in a manner that the vector can be expressed.

12. A method for producing a protein encoded by the polynucleotide according to claim 8 or 9, the method comprising culturing the transformant according to claim 11.

13. A method for dehydroxylating a hydroxyl group at a 9-position of urolithins, the method comprising step (I):
(I) bringing one or more selected from (i) to (iv) into contact with urolithins having a hydroxyl group at a 9-position to dehydroxylate the hydroxyl group at the 9-position:
(i) the enzyme according to any one of claims 1 to 7;
(ii) a protein encoded by the polynucleotide according to claim 8 or 9;
(iii) a microorganism producing the enzyme described in (i) above or the protein described in (ii) above; and
(iv) a treated product of the microorganism described in (iii) above.

14. The method according to claim 13, wherein
the urolithins are urolithin M5, urolithin M6, urolithin C, or isourolithin A; and
products produced by the dehydroxylation of the hydroxyl group at the 9-position of the urolithins are urolithin E, urolithin M7, urolithin A, and urolithin B, respectively.

15. A method for producing urolithin A, the method comprising steps (I) and (II):
(I) allowing a microorganism having an ability to produce urolithin C from ellagic acid to produce urolithin C from ellagic acid;
(II) bringing one or more selected from (i) to (iv) into contact with the urolithin C to produce urolithin A:
(i) the enzyme according to any one of claims 1 to 7;
(ii) a protein encoded by the polynucleotide according to claim 8 or 9;
(iii) a microorganism producing the enzyme described in (i) above or the protein described in (ii) above; and
(iv) a treated product of the microorganism described in (iii) above.

16. A method for producing urolithin B, the method comprising steps (I) to (III):
(I) allowing a microorganism having an ability to produce urolithin C from ellagic acid to produce urolithin C from ellagic acid;
(II) allowing a microorganism having an ability to produce isourolithin A from urolithin C to produce isourolithin A from the urolithin C; and
(III) bringing one or more selected from (i) to (iv) into contact with the isourolithin A to produce urolithin B:
(i) the enzyme according to any one of claims 1 to 7;
(ii) a protein encoded by the polynucleotide according to claim 8 or 9;
(iii) a microorganism producing the enzyme described in (i) above or the protein described in (ii) above; and
(iv) a treated product of the microorganism described in (iii) above.
